**Europäisches Patentamt**

**European Patent Office**

**Office européen des brevets**

(19)

(11) Veröffentlichungsnummer: **0 040 604**
**B1**

(12) **EUROPÄISCHE PATENTSCHRIFT**

(45) Veröffentlichungstag der Patentschrift:
29.05.85

(51) Int. Cl.⁴: **A 61 K 7/48**

(21) Anmeldenummer: **80901926.8**

(22) Anmeldetag: **17.10.80**

(86) Internationale Anmeldenummer:
**PCT/CH 80/00126**

(87) Internationale Veröffentlichungsnummer:
**WO 81/01514 (11.06.81 Gazette 81/14)**

(54) **KOSMETISCHE ZUBEREITUNG.**

(30) Priorität: **28.11.79 CH 10559/79**

(43) Veröffentlichungstag der Anmeldung:
**02.12.81 Patentblatt 81/48**

(45) Bekanntmachung des Hinweises auf die Patenterteilung:
**29.05.85 Patentblatt 85/22**

(84) Benannte Vertragsstaaten:
**FR**

(56) Entgegenhaltungen:
**DE - B - 1 300 620**
**FR - A - 873 553**
**FR - A - 1 390 184**
**FR - A - 1 433 383**
**US - A - 3 937 816**

**Die Akte enthält technische Angaben, die nach dem Eingang der Anmeldung eingereicht wurden und die nicht in dieser Patentschrift enthalten sind.**

(73) Patentinhaber: **Pentapharm A.G., Engelgasse 109, CH-4052 Basel (CH)**

(72) Erfinder: **RIES, Peter E., Unterer Rebbergweg 123, CH-4153 Reinach/BL (CH)**

(74) Vertreter: **Phélip, Bruno et al, c/o Cabinet Harlé & Phélip 21, rue de la Rochefoucauld, F-75009 Paris (FR)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft eine kosmetische Zubereitung, die als kosmetischen Wirkstoff einen wasserlöslichen Milzextrakt enthält.

Aus tierischen und menschlichen Organen hergestellte Wirkstoffe und deren Anwendung in der Kosmetik sind an sich bekannt. Als Kosmetikwirkstoffe werden insbesondere Placentaextrakte Ovarialextrakte und Hautextrakte verwendet. In den Placentaextrakten sind Enzyme und Hormone die eigentlichen Wirksubstanzen. In den Ovarialextrakten sind dies die Hormone, während die wirksamen Bestandteile der Hautextrakte in erster Linie die Hautproteine Kollagen und Elastin, d. h. hochmolekulare Proteine, sind.

In der französischen Patentschrift Nr. 1 390 184 ist u. a. die Verwendung eines kristallinen Milzextraktes als kosmetischer Wirkstoff beschrieben. Dieser in Wasser praktisch unlösliche Milzextrakt (hergestellt nach dem in der deutschen Patentschrift Nr. 1 109 833 beschriebenen Verfahren) enthält hauptsächlich lipidartige Inhaltsstoffe der Milz.

In der französischen Patentschrift Nr. 873 553 ist u. a. ein Verfahren zur Herstellung eines Milzextraktes beschrieben. Dieses Verfahren besteht darin, lebende Milzzellen mit einem langsam abbauend wirkenden Agens, z. B. Ultraviolettstrahlen oder Röntgenstrahlen, während längerer Zeit zu behandeln, ohne die genannten Zellen völlig zu zerstören, und das behandelte Zellmaterial zu extrahieren. Der Extrakt enthält die durch die genannte abbauende Behandlung gebildeten Wirkstoffe. Der erhaltene Extrakt wird als Kosmetikwirkstoff verwendet.

Es wurde nun gefunden, daß wäßrige Milzextrakte, die praktisch proteinfrei sind und wasserlösliche Peptide der Milz, aber keine lipidartigen Komponenten enthalten, auf die menschliche Haut eine überraschend günstige Wirkung ausüben und deshalb als Wirkstoffe in der Kosmetik verwendet werden können.

Wasserlösliche Milzextrakte und ihre Herstellung sind an sich bekannt und wurden von G. Ungar im Journ. Physiol., 36, 219 (1947), von Jackson und Temple (1966) im Australian Journal of Science und von D. M. Temple et al. in Comp. Biochem. Physiol., 17, 1089 (1966), beschrieben und zum Teil chemisch und biologisch charakterisiert. Die zuletzt genannten Autoren stellten sowohl aus Rindermilzen als auch aus Lebern durch Extraktion mit Wasser oder mit einer Mischung aus Wasser und einem wassermischbaren organischen Lösungsmitteln niedermolekulare wasserlösliche Extrakte her, die eine pharmakologische Wirkung, insbesondere eine inotrope Herzwirksamkeit hatten. Die oben angeführte Literatur enthält jedoch keinerlei Hinweise, die auf eine kosmetische Wirksamkeit der genannten Milzextrakte schließen lassen können.

Mit Hilfe einer entsprechenden elektronischen Meßapparatur, einem sog. »Oxygenmonitor« (siehe P. Eberhard et al., Medical and Biological Engineering, 5, 436—442 [1975]), konnte überraschenderweise nachgewiesen werden, daß der Sauerstoffpartialdruck in der menschlichen Haut durch die lokale Applikation eines wäßrigen Milzextraktes in Form einer kosmetischen Zubereitung deutlich erhöht wird.

Die verbesserte Sauerstoffversorgung der Hautzellen erhöht die regenerative Potenz der Haut und macht sie widerstandfähiger gegenüber den aggressiven Umwelteinflüssen des täglichen Lebens, die die Haut schneller altern lassen. Wasserlöslicher Milzextrakt ist somit ein geeigneter Kosmetikwirkstoff zur Regeneration der strapazierten und gealterten Haut.

Des weiteren konnte mit Hilfe der Fluvographie (siehe D. Braasch et al., Arzneimittel Forsch., 11, 102—104 [1961]), d. h. mit Mikrotemperaturfühlern gezeigt werden, daß durch die lokale Applikation von wasserlöslichem Milzextrakt die der Hautdurchblutung proportionale Wärmeleitzahl der Haut meßbar erhöht wird.

In Tierversuchen konnte gezeigt werden, daß sich Hautentzündungen, die durch Reizung der Haut mit Crotonöl, heißem Wasser oder durch Ultraviolettbestrahlung hervorgerufen wurden, durch Behandlung mit Externa, die wasserlöslichen Milzextrakt enthielten, schneller zurückbilden als mit Externa ohne Extraktzusatz. Diese Ergebnisse lassen die erwähnten Extrakte als geeignete Komponenten für Sonnenschutzkosmetika und sogenannte »After Sun«-Kosmetika erscheinen.

Pflegende Haarkosmetika in Form von Haarwässern, Haarspülungen und Shampoos wirken nicht nur auf das mehr oder weniger inerte Haar ein, sondern wirken zum großen Teil indirekt über eine Beeinflussung der Kopfhaut, mit der sie während der Behandlung in Berührung kommen. Es ist aus diesem Grunde zweckmäßig, wasserlöslichen Milzextrakt auch Haarkosmetika, die nicht zur Haarverformung oder -färbung eingesetzt werden, zuzusetzen.

Die vorliegende Erfindung betrifft eine kosmetische Zubereitung, die dadurch gekennzeichnet ist, daß sie als kosmetischen Wirkstoff einen durch wäßrige Extraktion von Schlachttiermilzen, Ausfällung der Proteine aus der wäßrigen Lösung mittels eines mit Wasser mischbaren organischen Lösungsmittels, Entfernung des organischen Lösungsmittels und gegebenenfalls Einengung der wäßrigen Lösung erhältlichen wasserlöslichen Milzextrakt und ein von reinem Wasser verschiedenes hautverträgliches Trägermaterial für den genannten Wirkstoff enthält.

Für den erfindungsgemäßen Zweck geeignete Milzextrakte können in an sich bekannter Weise wie folgt hergestellt werden: Frische, tiefgefrorene oder getrocknete Milzen von Schlachttieren werden entfettet, gemahlen und mit Wasser extrahiert. Der Extrakt wird durch Filtration oder Zentrifugation vom Geweberückstand abgetrennt und dann, zur Vermeidung von möglichen allergischen Reaktionen bei der späteren Anwendung, durch Fällung mittels eines mit Wasser mischbaren organischen Lösungsmittels enteiweißt und geklärt. Der so erhaltene wäßrige Extrakt kann zu einfacheren Handhabung eingeengt und zu einem Pulver aufgearbeitet werden. Durch Dialyse, Ultrafiltration, Gelfiltration, Chromatographie oder ähnliche Reinigungsverfahren kann der Extrakt weiter gereinigt und gegebenenfalls eingeengt oder bis zur Trockne konzentriert werden.

Zur Herstellung gebrauchsfertiger kosmetischer Zubereitungen hat sich die Verwendung einer 10%igen Lösung des getrockneten Extraktes in Wasser als günstig erwiesen. Diese 10%ige wäßrige Lösung ist klar, gelb bis braun, nicht viskos, praktisch eiweißfrei, hat eine Dichte von 1,030 bis 1,050 g/cm$^3$ und einen Brechungsindex von 1,300 bis 1,400. Diese Lösung dient als Ausgangsmaterial und kann in ein von reinem Wasser verschiedenes hautverträgliches Trägermaterial eingearbeitet werden.

Das aktive Prinzip des Milzextraktwirkstoffes ist chemisch noch nicht rein dargestellt worden. Das Molekulargewicht der im Extrakt enthaltenen wasserlöslichen Inhaltsstoffe liegt im Bereich von etwa 100 bis 10 000. Die Inhaltsstoffe des Extraktes sind thermostabil, so daß der letztere zur Verhinderung von Mikrobenwachstum durch Erhitzen sterilisiert werden kann. Er kann daher auch bei der Herstellung einer Creme oder Lotion der heißen wäßrigen Phase der Kosmetikformulierung zugesetzt werden.

Zur Herstellung von kosmetischen Zubereitungen wird der Milzextrakt einem kosmetischen Trägermaterial beigemischt. Als Trägermaterialien eignen sich wäßrige Alkohole, z. B. wäßriges Äthanol, Isopropanol, Propylenglykol, Glycerin oder Gemische davon; ferner Öl-in-Wasser- oder Wasser-in-Öl-Emulsionen und andere in der Kosmetik übliche Grundlagen. Bei Verwendung von wäßrigem Äthanol oder Isopropanol wird der Alkoholgehalt auf höchstens 70%, vorzugsweise auf 30% bis 50%, eingestellt. Das Mischungsverhältnis zwischen dem Extrakt und dem Trägermaterial wird zweckmäßig so eingestellt, daß die fertige kosmetische Zubereitung einen Gehalt von 0,05 bis 20%, vorzugsweise 0,1 bis 5% Extrakt, als Trockensubstanz berechnet, aufweist.

Die nachfolgenden Beispiele dienen zur Erläuterung der Erfindung.

## Beispiel 1

5 kg Rindermilzen wurden gemahlen und mit 10 Liter Wasser heiß extrahiert. Der Geweberückstand wurde durch Filtration unter Verwendung eines Filterhilfsmittels abgetrennt und nochmals mit 10 Liter Wasser heiß extrahiert. Die Extrakte der ersten und zweiten Extraktion wurden vereinigt und im Vakuum auf 1,3 Liter Lösung eingeengt, zur Enteiweißung mit 3,33 Liter 95%igem Alkohol versetzt und stehengelassen. Nach beendeter Eiweißpräzipitation wurde der wäßrig-alkoholische Extrakt klar filtriert, der Alkohol im Vakuum abgetrieben und der Extrakt durch Zugabe von Wasser und Konservierungsmittel auf einen Trockenrückstand von 10% eingestellt.

Zur Beurteilung der Aktivität des Extraktes wurde der folgende Test durchgeführt: In einer typischen Warburgversuchsanordnung ergab der Zusatz von 0,1 ml dieses enteiweißten 10%igen wäßrigen Milzextraktes zu 1 ml Meerschweinchenleberhomogenat eine Steigerung der Sauerstoffaufnahme von über 50% im Vergleich zum Leerwert ohne Zusatz von Milzextrakt.

## Beispiel 2

Ein nach Beispiel 1 hergestellter Milzextrakt wurde nach dem Abtreiben des Alkohols weiter bis zu einem Trockenrückstand von etwa 35% eingedampft, in einem Vakuumtrockenschrank zu einem bröckeligen Kuchen getrocknet und anschließend zu einem bräunlichgelben Pulver gemahlen. 10 g dieses Milzextraktpulvers wurden in 90 ml Wasser gelöst, mit p-Hydroxybenzoesäureester als Konservierungsmittel versetzt und filtriert. Die erhaltene klare hellbraune Milzextraktlösung zeigte im Warburgversuch volle Atmungsstimulation. Oxygenmonitoruntersuchungen auf der Haut am Menschen zeigten, daß nach einmaliger Behandlung der Haut mit dieser Milzextraktlösung der Sauerstoffpartialdruck der Haut nach einer Latenzzeit von etwa 30 Minuten um 6 bis 12 mm Hg anstieg und über längere Zeit anhielt.

## Beispiel 3

Eine nach Beispiel 2 hergestellte Milzextraktlösung wurde in eine kosmetische Zubereitung vom Typ einer O/W-Emulsionscreme folgender Zusammensetzung eingearbeitet:

3,0% Polyoxyäthylenglycerinmonostearat
2,0% Glycerinmono-di-stearat
3,0% Cetylalkohol

6,0% Stearinsäure
10,0% Isopropylmyristat
5,0% Fettsäuretriglycerid (Miglyol 812)
0,2% p-Hydroxybenzoesäureester als Konservierungsmittel (Nipagin)
2,0% Glycerin
2,0% Propylenglycol
0,3% Konservierungsmittel (Germall 115®)
10,0% wäßrige Milzextraktlösung, 10%ig
56,0% demineralisiertes Wasser
0,5% Parfüm

## Beispiel 4

Pflegende Hautlotion vom Typ O/W mit Zusatz von Milzextraktpulver:

3,0% Polyoxyäthylenglycerinmonostearat
2,0% Sorbitanfettsäureester
2,0% Cetylalkohol
8,0% Isopropylmyristat
0,2% p-Hydroxybenzoesäureester als Konservierungsmittel (Nipagin)
2,0% Propylenglycol
0,3% Konservierungsmittel (Germall 115®)
81,0% demineralisiertes Wasser
1,0% Milzextraktpulver
0,5% Parfüm

## Beispiel 5

»After Shave«-Lösung mit Milzextrakt:

50,0% Äthylalkohol, 96%ig
0,1% Methol
49,7% Wasser
0,2% Milzextraktpulver

## Beispiel 6

Haarwasser mit Milzextrakt:

45,0% Isopropanol
0,5% 1,2-Propylenglycol
0,5% Parfüm
51,0% Wasser
2,5% wäßrige Milzextraktlösung, 5%ig
0,5% Konservierungsmittel

## Patentansprüche

1. Kosmetische Zubereitung zur Pflege der Haut, dadurch gekennzeichnet, daß sie als kosmetischen Wirkstoff einen wasserlöslichen Milzextrakt, der die wasserlöslichen Milzinhaltsstoffe mit Molekulargewichten im Bereich von etwa 100 bis 10 000 enthält und praktisch proteinfrei ist, und ein von reinem Wasser verschiedenes hautverträgliches Trägermaterial für den genannten Wirkstoff enthält.

2. Kosmetische Zubereitung nach Patentanspruch 1, dadurch gekennzeichnet, daß sie als Wirkstoff einen durch wäßrige Extraktion von Schlachttiermilzen, Ausfällung der Proteine aus der wäßrigen Lösung mittels eines mit Wasser mischbaren organischen Lösungsmittels, Entfernung des organischen Lösungsmittels und gegebenenfalls Einengung der wäßrigen Lösung erhältlichen Milzextrakt enthält.

3. Kosmetische Zubereitung nach Patentanspruch 1, dadurch gekennzeichnet, daß sie als Trägermaterial wäßrigen Alkohol, eine Öl-in-Wasser- oder Wasser-in-Öl-Emulsion oder eine andere in der Kosmetik gebräuchliche Grundlage enthält.

4. Kosmetische Zubereitung nach den Patentansprüchen 1 und 3, dadurch gekennzeichnet, daß sie

4

einen Gehalt von 0,05 bis 20%, vorzugsweise 0,1 bis 5% des Wirkstoffes, als Trockensubstanz berechnet und bezogen auf das Gesamtgewicht der Zubereitung, aufweist.

## Claims

1. Cosmetic preparation for the care of the skin, characterized by the fact that it contains a water-soluble spleen extract as a cosmetically active principle, comprising the water-soluble spleen components with molecular weights of about 100 to 10,000, and being substantially protein-free, and a skin-compatible carrier different from pure water for the said active principle.

2. Cosmetic preparation according to claim 1, characterized by the fact that it contains as an active principle a spleen extract obtainable by aqueous extraction of spleens of slaughtered animals, precipitation of proteins from the aqueous solution by means of an organic solvent miscible with water, removal of the organic solvent and optionally concentration of the aqueous solution.

3. Cosmetic preparation according to claim 1, characterized by the fact that it contains as a carrier an aqueous alcohol, an oil-in-water or water-in-oil emulsion or another base commonly used in cosmetics.

4. Cosmetic preparation according to claims 1 and 3, characterized by the fact that its content of active principle is of 0.05 to 20%, preferably 0.1 to 5%, calculated as dry residue and based on the total weight of the preparation.

## Revendications

1. Préparation cosmétique pour les soins de la peau, caractérisée en ce qu'elle contient comme principe cosmétiquement actif un extrait de rate hydrosoluble qui comprend des composante hydrosolubles de la rate avec des poids moléculaires d'environ 100 à 10 000 et qui est pratiquement exempt de protéines, et en support pour ledit principe actif qui est bien toléré par la peau et différent de l'eau pure.

2. Préparation cosmétique selon la revendication 1, caractérisée en ce qu'elle contient comme principe actif un extrait de rate obtenu par extraction aqueuse de rates d'animaux de boucherie, précipitation des protéines de la solution aqueuse par un solvant organique miscible à l'eau, élimination du solvant organique et, le cas échéant, concentration de la solution aqueuse.

3, Préparation cosmétique selon la revendication 1, caractérisée en ce qu'elle contient comme support un alcool aqueux, une émulsion huile dans eau ou eau dans huile ou une autre base couramment utilisée en cosmétique.

4. Préparation cosmétique selon les revendications 1 et 3, caractérisée en ce qu'elle présente une teneur en principe actif de 0,05 à 20%, de préférence de 0,1 à 5%, calculée en tant que matière sèche et par rapport au poids total de la préparation.